# EUROPÄISCHE PATENTSCHRIFT

(11) **EP 0 412 191 B1**
(45) Veröffentlichungstag und Bekanntmachung des Hinweises auf die Patenterteilung: **03.02.1993**
(21) Anmeldenummer: 89114770.4
(22) Anmeldetag: 09.08.1989
(51) Int. Cl.: A61M 5/142, A61M 25/00

(54) **Implantierbarer Injektionskörper**
Implantable infusion device
Dispositif de perfusion implantable

(43) Veröffentlichungstag der Anmeldung: 13.02.1991
(73) Patentinhaber: SIEMENS AKTIENGESELLSCHAFT, 80333 München (DE)
(72) Erfinder: Hirschberg, Jakub, Dipl.-Ing., S-18344 Täby (SE); Svensson, Bengt, S-16242 Vällingby (SE)

(56) Entgegenhaltungen:
- WO-A-89/05671
- DE-A- 3 115 763
- DE-A- 3 641 107
- US-A- 4 055 178
- US-A- 4 306 563

## Beschreibung

Die Erfindung betrifft einen implantierbaren Injektionskörper für die zeitabhängig dosierbare Langzeitinjektion eines Medikamentes in einen lebenden Körper gemäß dem Oberbegriff des Patentanspruchs 1.

In vielen Fällen müssen flüssige Medikamente über längere Zeit appliziert werden. Für solche Langzeitapplikationen, wie sie beispielsweise bei Diabetikern erforderlich sind, stehen heute Medikamenten-Dosiergeräte zur Verfügung, die das flüssige Medikament, beispielsweise Insulin, in programmierbaren Dosierungen und Intervallen an den Patienten abgeben. Dabei kann es sich um externe, aber auch um in den Körper implantierbare Mikrodosiergeräte handeln. In beiden Fällen wird dem Patienten das Medikament über einen implantierten Injektionskörper zugeführt. Dies kann auf unterschiedliche Weise geschehen, nämlich subkutan, intraperitoneal oder intravenös. Im Fall der subkutanen Zuführung wird zwischen dem Ausgang der Medikamentenpumpe und dem Injektionsort ein Katheterschlauch subkutan verlegt, dessen distales Ende als Injektionsnadel aus Metall oder Plastik ausgebildet ist. Im Falle der intravenösen Injektion kann das distale Ende des Katheters stumpf enden oder eine spezielle Spitze aufweisen, die mit einer Austrittsöffnung für das Medikament versehen ist.

Ein solcher implantierbarer Injektionskörper in Katheterform ist beispielsweise in der DE-U-8 816 303 beschrieben. Solche Injektionssysteme werden innerhalb bestimmter Zeitspannen infolge von Fremdkörperreaktionen des Körpers angegriffen. Dabei lagert sich Bindegewebe von der Austrittsspitze beginnend in distaler Richtung an den Austrittskörper an. Darüber hinaus können Körperzellen, Makromoleküle und Gewebeteilchen in die Medikamentenaustrittsöffnungen eindringen und sich an den Innenwänden des Injektionskörpers anlagern. Durch diese Körperreaktionen kommt es letztlich zum Verschluß der Austrittsöffnung.

Um diesen Vorgang zu verzögern, ist die bekannte Anordnung mit einer von der Spitze in distaler Richtung versetzt angeordneten Austrittsöffnung versehen. Außerdem können bei dieser Anordnung distal von der Austrittsöffnung (Hauptaustrittsöffnung) versetzt angeordnete Durchtrittsschlitze als Notöffnungen vorgesehen werden, die bei verstopfter Hauptaustrittsöffnung und Verwendung plastischen Materials unter dem Druck des Medikamentes aufspreizen und einen Notaustritt ermöglichen. Da die Haftfähigkeit von Aufwachsungen in besonderem Maße von der Oberflächenrauhigkeit des Injektionskörpers und von dessen Oberflächengestalt abhängt, hat man solche Injektionskörper auch bereits mit besonders glatten und gewebeverträglichen Schutzschichten überzogen und die geometrische Form des Austrittskörpers so gewählt, daß Ecken und Kanten vermieden sind.

Alle diese Maßnahmen sind zwar geeignet, ein Zuwachsen oder Verstopfen der Medikamenten-Austrittsöffnung zu verzögern, können dies aber nicht verhindern. Auch das Ausweichen des Medikamentenaustritts auf Notöffnungen führt nicht zu einer störungsfreien Fortsetzung des Injektionsprozesses, sondern allenfalls dazu, daß es nicht zu einer sofortigen Unterbrechung der Medikamentenzufuhr kommt. Durch den höheren Flüssigkeitsdruck im gesamten Medikamententrakt als Folge des Austritts aus den schlitzförmigen Notöffnungen erhöht sich außerdem der Energieverbrauch der Pumpe, was zu einer Verkürzung der Lebensdauer der Energiequelle führt. Darüber hinaus besitzen die Schlitze der Notaustrittsöffnung relativ scharfe Kanten, was wiederum zu einer beschleunigten Anlagerung von Gewebeteilchen und damit zu einer relativ kurzen Standzeit einer solchen Öffnung führt. Auch die Anordnung mehrerer, gleichzeitig in Funktion befindlicher Öffnungen, führt nicht zum Ziel, weil sich die Durchtrittsgeschwindigkeit des Medikamentes, bezogen auf jede einzelne Öffnung, entsprechend der Anzahl der Öffnungen verringert. Nun ist aber erfahrungsgemäß die Standzeit einer Öffnung etwa proportional zur Durchtrittsgeschwindigkeit des Medikamentes. Je größer also die Durchtrittsgeschwindigkeit ist, um so höher ist auch die Standzeit einer solchen Öffnung. Mit den bisher bekannten Ausführungsformen von Katheter-Endstücken für die Injektion von Medikamenten war es jedenfalls nicht möglich, Standzeiten zu erreichen, die auch nur annähernd der Lebensdauer des Dosiergerätes entsprechen.

Der Erfindung liegt die Aufgabe zugrunde, die Standzeit des implantierbaren Injektionskörpers derart zu verlängern, daß sie wenigstens der Funktionsdauer des implantierten Dosiergerätes gleich ist.

Diese Aufgabe wird durch die im Patentanspruch 1 angegebene Erfindung gelöst. Dadurch ist erreicht, daß mehrere Öffnungen für den Medikamentenaustritt zur Verfügung stehen, von denen aber jeweils nur eine aktiv ist. Erst nach dem Verstopfen der jeweils aktiven Öffnung wird eine weitere freigegeben. Durch die Freigabe nur einer weiteren Öffnung anstelle der jeweils verstopften, bleibt die Durchflußgeschwindigkeit des Medikamentes etwa gleich und es können so viele Öffnungen vorgesehen werden, daß die Standzeit des Injektionskörpers nahezu beliebig verlängert werden kann. Damit erübrigen sich die bisher notwendigen mehrmaligen chirurgischen Eingriffe zum Auswechseln des Injektionskörpers auch bei noch funktionsfähigem Medikamenten-Dosiergerät.

In den Unteransprüchen sind Ausführungsformen für die verschiedenen Typen von Injektionskörpern beschrieben.

Dabei ist gemäß der Ausführungsform nach Patentanspruch 4 erreicht, daß die Dauer der Versiegelung der jeweiligen Austrittsöffnungen durch die unterschiedliche Dicke des Versiegelungsmaterials bereits vorgeprägt ist.

Gemäß Patentanspruch 5 kann die Auflösung des Versiegelungsmaterials allein durch biochemische Einflüsse der Körperumgebung erfolgen.

Gemäß Patentanspruch 6 ist diese Auflösung durch unterstützenden äußeren Einfluß, beispielsweise Ultraschalleinwirkung, auslösbar.

Gemäß Patentanspruch 7 kann dieser äußere Einfluß aber auch in einer erhöhten, von der normalen Medikamentenförderung unabhängigen Druckausübung, beispielsweise durch ein in ein zusätzliches Septum injiziertes Spülmittel erfolgen.

In einer weiteren Ausbildung der Erfindung gemäß Patentanspruch 9 besteht die Katheterspitze selbst in einer von der räumlichen Ausdehnung des Injektionsbereiches abhängigen Länge aus einem biochemisch oder durch äussere nichtinvasive Einwirkungen auflösbaren Material, so daß sich das Material, von der Katheterspitze beginnend, entgegen der Flußrichtung des Medikamentes auflöst, bevor eine Aufwachsung ein solches Aufmaß erreicht, daß die Katheterspitze verstopft. Die Katheterspitze weicht vielmehr einer möglichen Zuwachsung kontinuierlich aus. Auch dies kann durch unterstützendenäußeren Einfluß geschehen.

Weitere Einzelheiten der Erfindung werden anhand von Ausführungsbeispielen im folgenden näher erläutert. Darin zeigen:
Fig. 1 ein implantierbares Medikamenten-Dosiergerät mit an dessen Ausgang angeschlossenem Katheterschlauch und an dessen austrittsseitigem Ende angeordneter Katheterspitze,
Fig. 2 die Katheterspitze mit mehreren Medikamenten-Austrittsöffnungen und einer diese versiegelnden Ummantelung,
Fig. 3 eine andere Ausführungsform der Katheterspitze mit mehreren Medikamenten-Austrittsöffnungen unterschiedlicher Durchmesser, und
Fig. 4 ein Medikamenten-Dosiergerät mit direktem Austritt des Medikamentes über eine Vielzahl von zeitabhängig versiegelten Austrittsöffnungen, die in der Mantelfläche des Dosiergerätes in einer Reihe angeordnet.

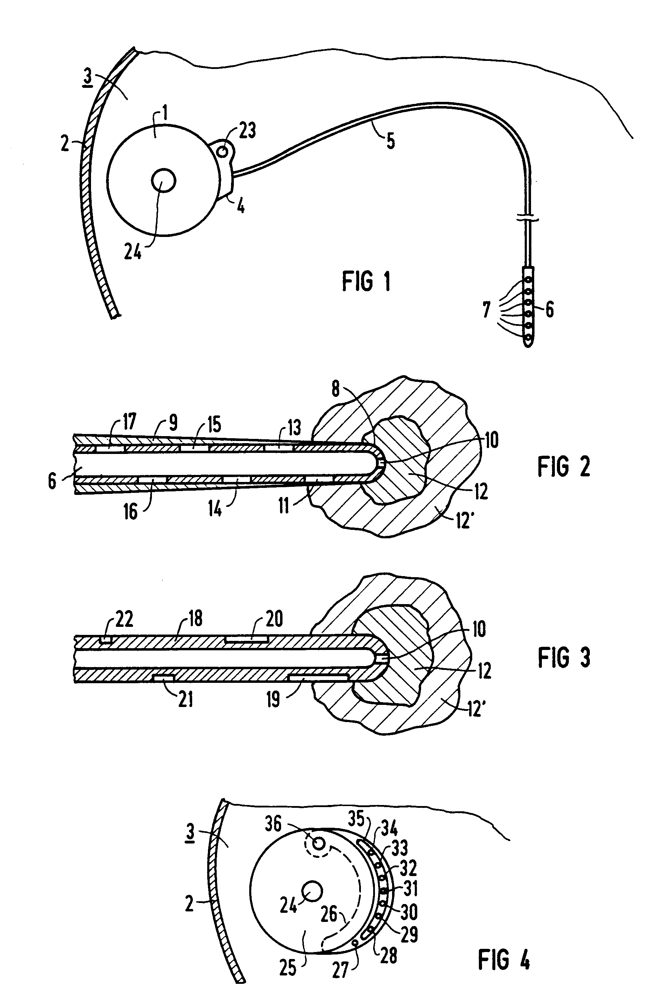

In Fig. 1 ist ein implantiertes Medikamenten-Dosiergerät 1 dargestellt, das unter der Haut 2 im Bauchraum 3 eines Patienten eingesetzt ist. Das Medikamenten-Dosiergerät 1 besitzt an seinem Medikamenten-Ausgang ein Katheter-Anschlußstück 4, das den Katheter 5 mit der nicht dargestellten Pumpe des Medikamenten-Dosiergerätes 1 verbindet.

Der Katheter 5, der beispielsweise in eine große Vene eingeführt sein kann, ist austrittsseitig mit einer Medikamenten-Austrittskanüle 6 versehen, welche eine Anzahl von in Fließrichtung des Medikamentes aufeinanderfolgenden Medikamenten-Austrittsöffnungen 7 besitzt.

Wie bereits erwähnt, ist der Körper bestrebt, den als Fremdkörper empfundenen Katheter 5 mit Bindegewebe zu umwachsen, so daß die Medikamenten-Austrittsöffnung blockiert wird. Eine solche Blockade kann auch dadurch auftreten, daß sich Fremdpartikel oder auskristallisiertes Medikament an die Öffnung anlagern und diese dadurch verstopfen. Bei intravenöser Katheterlage kann eine solche Verstopfung auch durch koagulierte Blutpartikel auftreten. In einem solchen Falle muß chirurgisch eingegriffen und der Katheter ausgewechselt werden. Dies ist ein den Patienten belastender Eingriff, der in vielen Fällen bereits lange vor dem Ende der Funktionsdauer des implantierten Medikamenten-Dosiergerätes, etwa bei Erreichen des Endes der Batterie-Lebensdauer, erforderlich wird. Im Falle einer durch drahtlose Energietransformation wieder aufladbaren Batterie kann von einer noch weit höheren Lebensdauer des Medikamenten-Dosiergerätes ausgegangen werden. Somit werden die Abstände der chirurgischen Eingriffe zur Auswechslung des Implantates im wesentlichen von der Öffnungsdauer der als Injektionskörper verwendeten Medikamenten-Austrittskanüle 6 bestimmt. Wenn diese Kanüle, wie in Fig. 1 dargestellt, mit mehreren in Fließrichtung des Medikamentes aufeinanderfolgenden und in einem räumlichen Abstand voneinander angeordneten Medikamenten-Austrittsöffnungen 7 versehen wird, ist die Fließgeschwindigkeit des Medikamentes, bezogen auf jede einzelne Öffnung, sehr gering. Nachdem die Zuwachsgeschwindigkeit jeder einzelnen Öffnung auch von der Durchtrittsgeschwindigkeit des Medikamentes abhängt, wird das Zuwachsen jeder einzelnen Medikamenten-Austrittsöffnung durch diese Maßnahme noch erhöht, so daß sich der durch die Vielzahl von Öffnungen zunächst vorhandene Vorteil wieder sehr stark verringert.

In Fig. 2 ist dargestellt, wie dieser Nachteil einer verringerten Fließgeschwindigkeit des Medikamentes auch bei einer großen Anzahl von Öffnungen erfindungsgemäß vermieden werden kann. Dies geschieht dadurch, daß alle Austrittsöffnungen bis auf eine einzige zunächst durch einen Überzug aus Versiegelungsmaterial 9 verschlossen sind. Nur die primäre Medikamenten-Austrittsöffnung 10 in der Kanülenspitze 8 ist geöffnet. Als Versiegelungsmaterial ist beispielsweise ein biologisch verträglicher Polyester verwendbar, der demjenigen entspricht, der etwa auch für selbstauflösendes Nahtmaterial in der Chirurgie verwendet wird. Es ist auch möglich, ein Biogel, beispielsweise Hydroxybutyrat oder Hydroxyvelenat als Versiegelungsmaterial zu verwenden. Einige Zeit nach der Implantation des Katheters beginnt der Überwachsungsprozeß. Gleichzeitig beginnt aber auch der Auflösungsprozeß des Versiegelungsmaterials 9. Wenn nun dieses Material nicht mit konstanter Wandstärke, sondern mit entgegen der Fließrichtung des Medikamentes kontinuierlich steigender Wandstärke aufgetragen ist, öffnet sich zunächst die der primären Medikamenten-Austrittsöffnung 10 nächstliegende versiegelte Austrittsöffnung 11, was im Idealfalle genau zu dem Zeitpunkt geschieht, zu dem die primäre Austrittsöffnung 10 durch die Aufwachsung 12 verschließt.

Aufgrund der steigenden Wandstärke des Versiegelungsmaterials 9 in Richtung gegen den Medikamentenfluß wird sich dieser Vorgang bei den folgenden versiegelten Austrittsöffnungen 13-17 sukzessive fortsetzen. Wenn also die Aufwachsung die mit 12′ bezeichnete Ausdehnung erreicht hat, wird die Austrittsöffnung 11 verschlossen. Dieser Vorgang wiederholt sich in aufsteigender Folge, bis schließlich die letzte versiegelte Austrittsöffnung 17 ihre Funktion beendet. Je nach zur Verfügung stehender räumlicher Ausdehnung des Injektionsbereiches kann nun die Anzahl der versiegelten Medikamenten-Austrittsöffnungen derart groß gewählt werden, daß die Dauer bis zum Zuwachsen der in Fließrichtung des Medikamentes letzten Öffnung etwa die Standzeit des Medikamenten-Dosiergerätes erreicht.

Nun ist die Geschwindigkeit der Aufwachsung und damit der Abstand zwischen dem Schließen der einzelnen Medikamentenöffnungen eine individuelle Größe, die stark vom statistischen Mittelwert abweichen kann. Es kann daher zweckmäßig sein, die Freigabe von versiegelten Austrittsöffnungen durch äußere Einwirkung zu beeinflussen oder auch alle in zu verursachen. Eine solche äußere Einflußnahme kann beispielsweise mit Hilfe eines nach bildgebender Lokalisierung auf das jeweils zu öffnende Loch einwirkenden gebündelten Ultraschallstrahles erfolgen.

Eine andere Möglichkeit der Einflußnahme zum Durchstoßen der jeweils folgenden versiegelten Austrittsöffnung ist in Fig. 3 dargestellt. Die Medikamenten-Austrittskanüle 18 ist mit nicht durchgehenden Bohrungen als späteren Medikamenten-Austrittsöffnungen unterschiedlichen Durchmessers versehen. Dabei hat das verbleibende, die Öffnung zunächst verschließende Restmaterial Membranstärke. Die der primären offenen Austrittsöffnung 10 gegen die Fließrichtung des Medikamentes nächstliegende, zunächst geschlossene Austrittsöffnung 19 besitzt einen relativ zur primären Austrittsöffnung 10 großen Durchmesser. Dieser Öffnungsdurchmesser verringert sich bei den weiteren, zunächst geschlossenen Austrittsöffnungen 20, 21, 22 um jeweils bestimmte Beträge. Die zunächst geschlossenen Austrittsöffnungen 19-22 können auch durchgehend gebohrt und durch ein Versiegelungsmaterial zunächst verschlossen sein. Dabei kann es sich ebenfalls um ein Material handeln, welches von der umgebenden Körperflüssigkeit allmählich aufgelöst wird. In diesem Fall muß die Materialstärke des Versiegelungsmaterials mit geringer werdenden Durchmessern der Medikamenten-Austrittsöffnungen steigen. Die absolute Materialstärke des Versiegelungsmaterials muß dabei so groß gewählt werden, daß bei individuell relativ langsamer Zersetzung die Austrittsöffnungen nicht vorzeitig entsiegelt werden. Diese Entsiegelung kann so vor sich gehen, daß bei Zuwachsen beispielsweise der primären Austrittsöffnung 10 durch den dann ansteigenden Flüssigkeitsdruck des Medikamentes die Restmembran der Austrittsöffnung 19 wegen ihres großen Durchmessers und des dadurch höheren Flächendrucks bei einem niedrigeren absoluten Druck als derjenige, der für die anderen Austrittsöffnungen notwendig wäre, geöffnet wird. Die Öffnung kann aber auch auf andere Weise dadurch erfolgen, daß dem Katheter-Anschlußstück 4 ein Spülseptum 23 (Fig. 1) zugeordnet wird, in welches eine Spülflüssigkeit von außen injiziert werden kann, die über eine nicht dargestellte Ventilanordnung gegen den über ein Medikamenten-Nachfüllseptum 24 nachfüllbaren, nicht dargestellten Medikamenten-Vorratsbehälter gesperrt und gegenüber dem Katheter 5 geöffnet ist. Damit kann der Katheterdruck soweit erhöht werden, daß im Falle einer Zuwachsung einer Medikamentenöffnung die Membran der jeweils nächsten Öffnung durchlässig wird.

In Fig. 4 ist eine weitere Ausführungsform eines im Bauchraum 3 eines Patienten implantierten Medikamenten-Dosiergerätes 25 dargestellt, bei dem der Injektionskörper Bestandteil des Dosiergerätes selbst ist. Das Dosiergerät ist mit einer nur andeutungsweise dargestellten peripheren Medikamenten-Austrittskammer 26 ausgestattet, die intern mit dem Ausgang der nicht dargestellten Dosierpumpe verbunden und nach außen über eine Reihe von Medikamenten-Austrittsöffnungen 27-34 durch die Wand des Dosiergerätes geöffnet ist. Dabei ist zunächst nur die Austrittsöffnung 27 frei, während die Austrittsöffnungen 28-34 mit Versiegelungsmaterial 35 abgedeckt sind. Dabei kann das Versiegelungsmaterial 35 in ähnlicher Weise, wie bei der Medikamenten-Austrittskanüle gemäß Fig. 2, von der Austrittsöffnung 28 beginnend bis zur Austrittsöffnung 34 eine stetig ansteigende Dicke besitzen, es kann aber auch analog zu der Medikamenten-Austrittskanüle gemäß Fig. 3 der Lochdurchmesser der Austrittsöffnungen beginnend mit einem relativ großen Durchmesser der Öffnung 28 kontinuierlich bis zur Öffnung 34 abfallend gewählt werden. Die Austrittsöffnungen 28-34 werden dann ähnlich wie in Fig. 3 dargestellt mit einem Versiegelungsmaterial geschlossen, welches ebenfalls in seiner Stärke von der Austrittsöffnung 28 beginnend bis zur Austrittsöffnung 34 kontinuierlich zunehmen kann, so daß bei einem durch biochemische Einflüsse erfolgenden Materialabbau die Austrittsöffnungen nacheinander für den Durchtritt des Medikamentes vorbereitet werden. Die endgültige Öffnung kann dann mit Hilfe eines weiteren, in die Medikamenten-Austrittskammer 26 mündenden Spülseptums 36 unter dem Druck des Spülmittels erfolgen.

## Patentansprüche

1. Implantierbarer Injektionskörper für die zeitabhängig dosierbare Langzeitinjektion eines Medikamentes in einen lebenden Körper, das in einem implantierten Medikamenten-Dosiergerät gespeichert und von diesem mittels einer programmierbaren Pumpenanordnung dem Injektionskörper zuführbar ist, der mit einer Anzahl von in Fließrichtung des Medikamentes aufeinanderfolgenden und in einem räumlichen Abstand voneinander angeordneten Medikamenten-Austrittsöffnungen versehen ist, **dadurch gekennzeichnet,** daß die einzelnen Austrittsöffnungen (11-17, 19-22 und 28-34) bis auf eine (10, 27) mit Hilfe eines zu unterschiedlichen Zeitpunkten auflösbaren, gewebeverträglichen plastischen Materials (7, 18, 35) derart flüssigkeitsdicht versiegelt sind, daß nach dem durch Umgebungseinflüsse verursachten Verschließen der jeweils offenen Austrittsöffnung eine der versiegelten Austrittsöffnungen durch chemisches oder physikalisches Entfernen des Versiegelungsmaterials für den Medikamentendurchfluß freigebbar ist.

2. Injektionskörper nach Anspruch 1, **dadurch gekennzeichnet,** daß dieser als am distalen Ende eines Katheters (5) angeordnete Injektionskanüle (6, 18) ausgebildet ist, deren Spitze mit einer freien Austrittsöffnung (10) und mit einer Anzahl von jeweils gegen die Fließrichtung des Medikamentes versetzt angeordneten versiegelten Austrittsöffnungen (11-17, 19-22) versehen ist.

3. Injektionskörper nach Anspruch 1, **dadurch gekennzeichnet,** daß dieser Teil des implantierten Medikamenten-Dosiergerätes (25) selbst ist, in der Weise, daß die Medikamenten-Austrittsöffnungen (27-34) als Durchbrüche durch das Gehäuse des Medikamenten-Dosiergerätes (25) ausgebildet sind.

4. Injektionskörper nach Anspruch 1, 2 oder 3, **dadurch gekennzeichnet**, daß als Versiegelungsmaterial gewebeverträglicher plastischer Kunststoff (9) benutzt ist und die einzelnen Öffnungen (11-17) mit diesem Kunststoff in unterschiedlicher, vom jeweils notwendigen Offnungszeitpunkt abhängigen Dicke versiegelt sind.

5. Injektionskörper nach Anspruch 4, **dadurch** **gekennzeichnet**, daß ein durch biochemische Umgebungseinflüsse innerhalb einer von seiner Dicke abhängigen Zeitspanne sich auflösender Kunststoff verwendet ist.

6. Injektionskörper nach Anspruch 4, **dadurch gekennzeichnet,** daß ein durch nicht invasive thermische oder chemische äußere Einwirkung auflösbarer Kunststoff verwendet ist.

7. Injektionskörper nach Anspruch 4, **dadurch gekennzeichnet**, daß dieser mit einem von außerhalb des Körpers erreichbaren Einstichseptum (23, 36) versehen ist, in das ein Spülmittel unter einem solchen Druck injizierbar ist, daß bei verschlossener aktiver Öffnung die jeweils folgende versiegelte Öffnung geöffnet wird.

8. Injektionskörper nach Anspruch 7, **dadurch gekennzeichnet**, daß die Öffnungen (19-22), beginnend mit der der freien Austrittsöffnung (10) nächstliegenden versiegelten Öffnung (19), mit zunehmendem Abstand von dieser einen steigenden Durchmesser aufweisen.

9. Implantierbarer Injektionskörper in Form eines Katheters für die zeitabhängige dosierbare Langzeitinjektion eines Medikaments in einen lebenden Körper, das in einem implantierten Medikamenten-Dosiergerät gespeichert und von diesem mittels einer programmierbaren Pumpenanordnung dem Injektionskörper zuführbar ist, der an der Katheterspitze eine Austrittsöffnung für das Medikament aufweist, **dadurch gekennzeichnet**, daß die Katheterspitze in einer von der räumlichen Ausdehnung des Injektionsbereiches abhängigen Länge aus einem biochemisch oder durch äußere nichtinvasive Einwirkung auflösbaren Material besteht, so daß sich das Material, von der Katheterspitze beginnend, entgegen der Flußrichtung des Medikamentes auflöst und damit die Katheterspitze einer möglichen Verstopfung durch Umgebungseinflüsse kontinuierlich ausweicht.

10. Injektionskörper nach Anspruch 9, **dadurch** **gekennzeichnet**, daß der Materialquerschnitt des auflösbaren Materials entgegen der Fließrichtung des Medikamentes zunimmt.

## Claims

1. Implantable infusion device for the long-term infusion, which can be metered time-dependently, of a medicament into a living body, which medicament is stored in an implanted medicament metering unit and can be delivered by the latter by means of a programmable pump arrangement to the infusion device which is provided with a number of medicament outlet openings which are arranged successively in the flow direction of the medicament and with spacing from one another, characterised in that the individual outlet openings (11-17, 19-22 and 28-34), with the exception of one (10, 27), are fluid-tightly sealed with the aid of a histocompatible plastic material (7, 18, 35) which can be dissolved at different points in time in such a way that, after closure of the respectively open outlet opening caused by environmental influences, one of the sealed outlet openings can be opened up by chemical or physical removal of the sealing material for the throughflow of the medicament.

2. Infusion device according to Claim 1, characterised in that it is constructed as an infusion cannula (6, 18) which is arranged at the distal end of a catheter (5) and whose tip is provided with a free outlet opening (10) and with a number of sealed outlet openings (11-17, 19-22) arranged offset in each case counter to the flow direction of the medicament.

3. Infusion device according to Claim 1, characterised in that it is part of the implanted medicament metering unit (25) itself in such a way that the medicament outlet openings (27-34) are constructed as passages through the housing of the medicament metering unit (25).

4. Infusion device according to Claim 1, 2 or 3, characterised in that histocompatible plastic synthetic material (9) is used as sealing arterial and the individual openings (11-17) are sealed by this synthetic material with a differing thickness which is dependent on the point in time of opening required in each case.

5. Infusion device according to Claim 4, characterised in that a synthetic material is used which dissolves due to biochemical environmental influences within a period of time depending on its thickness.

6. Infusion device according to Claim 4, characterised in that a synthetic material is used which can be dissolved by non-invasive external theramal or chemical action.

7. Infusion device according to Claim 4, characterised in that it is provided with a puncture septum (23, 36) which can be reached from outside the body and into which a rinsing agent can be infused at such a pressure that, in the case of a closed active opening, the respective following sealed opening is opened.

8. Infusion device according to Claim 7, characterised in that, beginning with the sealed opening (19) nearest to the free outlet opening (10), the openings (19-22) have an increasing diameter with increasing spacing from said sealed opening (19).

9. Implantable infusion device in the form of a catheter for the long-term infusion, which can be metered time-dependently, of a medicament into a living body, which medicament is stored in an implanted medicament metering unit and can be delivered by the latter by means of a programmable pump arrangement to the infusion device which has an outlet opening for the medicament at the catheter tip, characterised in that the catheter tip consists of a material, which can be dissolved biochemically or by external, non-invasive action, over a length depending on the spatial extent of the infusion area, such that the material, beginning at the catheter tip, dissolves counter to the flow direction of the medicament, and the catheter tip thus continuously escapes possible blockage due to environmental influences.

10. Infusion device according to Claim 9, characterised in that the material cross-section of the soluble material increases counter to the flow direction of the medicament.

## Revendications

1. Dispositif d'injection implantable pour l'injection de longue durée, dosable en fonction du temps, d'un médicament dans un corps vivant, ce médicament étant stocké dans un appareil implanté de dosage du médicament et pouvant être envoyé par ce dernier, au moyen d'un dispositif de pompe programmable, au dispositif d'injection, qui est pourvu d'un nombre d'ouvertures de sortie du médicament, qui se succèdent dans la direction d'écoulement du médicament et sont spatialement distantes les unes des autres, caractérisé par le fait que les différentes ouvertures de sortie (11-17,19-22 et 28-34) peuvent être fermées d'une manière étanche aux liquides, hormis l'une d'elles (10,27), à l'aide d'une matière plastique (16,18,35) compatible avec le tissu et pouvant être dissoute à des instants différents, de telle sorte qu'après l'obturation, provoquée par des influences de l'environnement, de l'ouverture de sortie respectivement ouverte, l'une des ouvertures de sortie obturées peut, pour le passage du médicament, être dégagée au moyen d'une élimination chimique ou physique du matériau d'obturation.

2. Dispositif d'injection suivant la revendication 1, caractérisé par le fait que ce dernier est agencé sous la forme d'une canule d'injection (6,18), qui est disposée sur l'extrémité distale d'un cathéter et dont la pointe est pourvue d'une ouverture libre de sortie (10) et d'un nombre d'ouvertures de sortie obturées (11-17, 19-22), qui sont disposées en étant décalées respectivement dans le sens opposé de la direction d'écoulement du médicament.

3. Dispositif d'injection suivant la revendication 1, caractérisé par le fait que ce dispositif fait partie de l'appareil implanté (25) de dosage du médicament, lui-même en ce sens que les ouvertures (27-34) de sortie du médicament sont réalisées sous la forme de passages traversant le boîtier de l'appareil (25) de dosage du médicament.

4. Dispositif d'injection suivant la revendication 1, 2 ou 3, caractérisé par le fait qu'on utilise, comme matériau d'obturation, une matière plastique (9) compatible avec le tissu et que les différentes ouvertures (11-17) sont obturées par cette matière plastique avec une épaisseur variable, qui dépend de l'instant respectif requis d'ouverture.

5. Dispositif d'injection suivant la revendication 4, caractérisé par le fait qu'on utilise une matière plastique qui est dissoute mous l'effet d'influences biochimiques de l'environnement en un intervalle de temps qui dépend de son épaisseur.

6. Dispositif d'injection suivant la revendication 4, caractérisé par le fait qu'on utilise une matière plastique qui est dissoute sous l'effet d'une influence extérieure thermique ou chimique, non invasive.

7. Dispositif d'injection suivant la revendication 4, caractérisé par le fait que ce dernier est pourvu d'une membrane perforable (23, 36), que l'on peut atteindre de l'extérieur du corps et dans laquelle on peut injecter un agent de lavage à une pression telle que, lorsque l'ouverture active est fermée, l'ouverture obturée suivante s'ouvre.

8. Dispositif d'injection suivant la revendication 7, caractérisé par le fait que les ouvertures (19-22) possèdent, en commençant par l'ouverture obturée (19) qui est la plus proche de l'ouverture libre de sortie (17), un diamètre qui augmente au fur et à mesure qu'on s'écarte de cette ouverture.

9. Dispositif d'injection implantable sous la forme d'un cathéter pour l'injection le longue durée, pouvant être dosée en fonction du temps, d'un médicament dans un corps vivant, médicament qui est stocké dans un appareil implanté de dosage du médicament et peut être envoyé par ce dernier, au moyen d'un dispositif de pompe programmable, au dispositif d'injection, qui possède une ouverture de sortie pour le médicament au niveau de la pointe d'un cathéter, caractérisé par le fait que la pointe du cathéter est réalisée avec une longueur, qui dépend de l'étendue spatiale de la zone d'injection, d'un matériau pouvant être dissous par voie biochimique ou au moyen d'une action extérieure non invasive, de sorte que le matériau de dissout, en partant de la pointe du cathéter, dans le sens opposé de la direction d'écoulement du médicament et que par conséquent la pointe du cathéter est libérée en permanence d'une obturation possible due à des influences de l'environnement.

10. Dispositif d'injection suivant la revendication 9, caractérisé par le fait que la section transversale du matériau apte à se dissoudre augmente en sens opposé de la direction d'écoulement du médicament.
